# EUROPEAN PATENT SPECIFICATION

(11) **EP 2 185 205 B1**
(45) Date of publication and mention of the grant of the patent: **24.07.2013**
(21) Application number: 08788437.5
(22) Date of filing: 22.08.2008
(51) Int. Cl.: A61L 9/04, A61L 9/12, A01M 1/20

(54) **PASSIVE DISPENSER OF VOLATILE MATERIAL**
PASSIVER SPENDER VON FLÜCHTIGEM MATERIAL
DISTRIBUTEUR PASSIF DE MATÉRIAU VOLATILE

(30) Priority: 23.08.2007 GB 0716412
(43) Date of publication of application: 19.05.2010
(73) Proprietor: Reckitt & Colman (Overseas) Limited, Slough Berkshire SL1 3UH (GB)
(72) Inventor: DUDDINGTON, Andrea, Hull HU8 7DS (GB); HINDLE, Benjamin, David, Hull HU8 7DS (GB)
(74) Representative: Carlin, Robert George
(86) International application number: PCT/GB2008/002878
(87) International publication number: WO 2009/024802

(56) References cited:
- US-A- 4 226 829
- US-A- 4 889 286
- US-A1- 2006 233 538
- US-A1- 2007 176 015

## Description

### Field of the Invention

The present invention relates to a dispenser adapted to passively emanate a volatile material at room temperatures via a membrane, and particularly but not exclusively, to emanate a volatile material such as a fragrance/air freshener, an insecticide, a disinfectant, a bactericide, a fungicide and/or a medicament.

### Background

Conventional membrane-containing emanation devices, such as devices found for the emanation of volatile liquid air fresheners, generally consist of a housing holding a quantity of the liquid air freshener therein and a membrane in constant contact on one side thereof with the liquid and exposed to the external environment on its other side. The contact between the volatile liquid and membrane causes the membrane to uptake the liquid by capillary action and/or diffusion, thus permitting the emanation of the liquid by evaporation from its surface exposed to the environment.

Whilst such devices are simple to construct they contain several drawbacks, some of which are identified below.

One drawback, particularly with the emanation of air fresheners is that of a phenomenon called "habituation". Habituation is when users of a continuously emanating air freshener get so used to the fragrance of the air freshener that they become unable to notice it after a period of time. This is problematic in particular with non-automated air freshening devices where the user is given no visual clues as to whether the device is emanating a fragrance or not.

A further drawback associated with devices having a membrane in constant contact with a volatile material, such as a volatile liquid air freshener, is the phenomena of vacuum build-up. The vacuum build-up phenomena can occur inside the housing due to the wetted membrane being unable to allow sufficient flow of air therethrough in order to equalise the drop in pressure inside the housing caused by the emanation of the volatile material. This build-up phenomena can cause unacceptable performance problems with such a device from a consumer perspective.

Known technologies within the field of the present invention are described in US4226829 in which an air treatment device is described having an appearance similar to an hourglass wherein each end of the hourglass is covered by a porous membrane and the air treatment agent can be manually moved between chambers of the hourglass by inversion. US4889286, in contrast to US'829, describes an air treatment device which is equivalent to half an hourglass in which a bottle can possess an operating position in which the bottle chamber is inverted such that the bottle opening, which is covered by a membrane, is located below the main chamber of the bottle which also contains air treatment material which impregnates the membrane and emanates therethrough, whereas the device is 'turned off' by turning the bottle to a non-inverted position which removes the air treatment material from direct contact with the membrane and thus ceases the emanation thereof. US2006/0233538 describes a variation on the hourglass theme wherein the two chambers of the hourglass hold liquid air treatment agent and are connected by a porous wick which allows liquid transport therealong to a central uncovered portion of the wick from where emanation can occur. Also, W02006/128316 describes a slightly different device which is a simple container having a single opening covered by a permeable membrane which can be brought into contact with a quantity of volatile air treatment agent held within the container to emanate same.

In the field of air freshening it is generally preferred to use a volatile liquid fragrance/air freshener comprising several components. These components often possess different volatilities which can lead to emission problems. In devices having a membrane in constant contact with the volatile liquid, an accumulation of volatile liquid components with the lowest comparative volatilities can occur leading to an undesirable non-uniform emanation profile for the liquid.

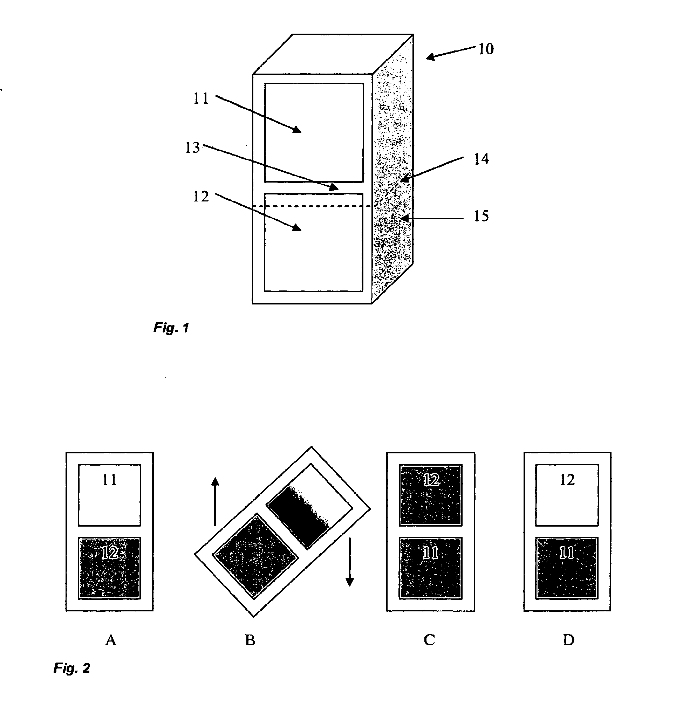

In either or both cases of build-up phenomena and accumulation of low volatility components, the evaporation rate, and thus the emanation rate, of the volatile liquid adopts the profile shown in Fig. 1 below.

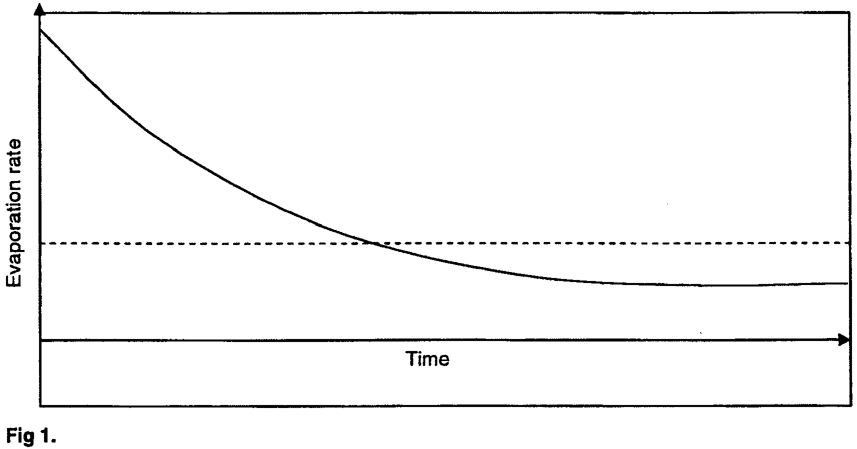

Eventually the emanation rate may reach equilibrium (dashed line) where the rate of evaporation of each individual component of the volatile liquid away from the membrane surface is equivalent to the rate of deposition due to diffusion and the device cannot maintain any further vacuum.

Accordingly, it is an object of the present invention to provide a device that is capable of addressing the abovementioned drawbacks and other drawbacks that will be appreciated by a person skilled in the art.

### Summary of invention

According to a first aspect of the present invention therefore, there is provided a passive dispenser for volatile material as defined by claim 1.

According to an embodiment of the present invention the volatile material is provided in said chamber.

In the context of the present invention, the term "passive" is understood to mean the ability to emanate a volatile liquid without the assistance of electrically powered emanation means or propellant-powered aerosol systems or the like.

The membrane(s) not in contact with the volatile material may advantageously provide the dispensers according to the present invention with the ability to at least overcome the build-up phenomena associated with the prior art.

Furthermore, and as will be discussed below, the additional drawbacks of habituation and avoiding the accumulation of low volatility components may also be advantageously addressed by the dispensers according to the present invention.

The housing of the dispenser preferably comprises a front wall and a rear wall spaced apart by side edges, or a side edge where the housing is of a generally circular or elliptical shape when viewed from a side, thus defining the inner chamber. In one preferred embodiment the housing is provided in a generally circular or elliptical shape when viewed from a side. In an alternatively preferred embodiment the housing is provided, when viewed from a side, with a substantially regular polygon shape such as a triangle, a rectangle, a pentagon, a hexagon, a heptagon, or an octagon. In a further alternatively preferred embodiment the housing is provided, when viewed from a side, with a substantially regular polygon shape such as a star, a quadrilateral shape, or parallelogram shape.

The housing may be made from any suitable rigid, resilient or substantially resilient material; a plastics material is particularly preferred.

The membranes are attached to the housing, preferably to the front face thereof, such that one side of each membrane is exposed to the inner chamber of the housing and the other side of the membrane is exposed to the exterior of the housing.

Alternatively, said membranes may be disposed on the same or different walls or side(s) thereof, providing that, in use, the dispenser may be orientated such that at least one of said membranes is not in contact with the volatile material contained within the inner chamber.

The membranes may be attached such that they each form a leak-proof seal with the housing to prevent leaking of any volatile material through the seal when the dispenser is in use. The leak-proof seals may be particularly useful in preventing or minimising leaking of the volatile material when the dispenser has been knocked over or placed with the membrane(s) facing downwardly toward a surface on which the dispenser has been placed.

A preferred operating position is achieved where the dispenser is configured such that, in use, the membranes are not facing toward the surface on which the dispenser is placed. Even more preferably the dispenser is configured such that, in use, the membranes are facing in a substantially perpendicular direction to the surface on which the dispenser is placed.

The dispenser may be configured such that, in use, the preferred operating position is for the side edge or edges of the housing to contact the surface on which the dispenser is placed.

The housing may have one or more securing means protruding from or mounted on the exterior thereof. Said securing means may be adapted to engage the surface on which the dispenser is placed in order to secure it, in use, in one or more desired orientations with respect to the membranes and volatile material. Preferably the housing is provided with a plurality of securing means to permit the dispenser to be positioned a variety of orientations.

A barrier section is preferably located between adjacent membranes to prevent against uptake of volatile material, in use, by one membrane from an adjacent membrane or membranes. The barrier section may be provided by the structure or the housing and/or by suitable treatment of the membranes to prevent lateral uptake of the volatile material in use.

In preferred embodiments of the invention, the volatile material is present in a quantity in the dispenser that, when in use and the dispenser is orientated in a preferred operating position, the volatile material does not come into direct contact with the entirety of a discrete membrane. The precise provision of a specific quantity of volatile material in order to not contact the entirety of a discrete membrane is considered to be advantageous with respect to the ability of overcoming the build-up phenomena associated with the prior art.

The membranes are preferably constructed from a material that is vapour-permeable. Alternatively the membranes may be constructed from a material that is non-vapour-permeable. The selection of the particular properties of the membranes may alter depending on the particular volatile material the dispenser intends to emanate when in use. For instance, where the volatile material is a blend of components having greatly differing volatilities, it may be preferable to use a non-vapour-permeable membrane to provide a more consistent evaporation profile of the material.

The membranes are preferably made from one or more polymeric materials or polymeric materials in combination with inorganic materials, for example silica. Suitable polymeric materials may include but are not limited to polyethylene, polypropylene, polystyrene and or copolymers of the aforesaid and/or mixtures of any or all of the aforesaid materials.

The polymeric material used in the membranes of the present invention may further incorporate suitable components such as: fillers; plasticizers; antioxidants; lubricants; anti-statics; pigments; dyestuffs; stabilizers; light stabilizers; non-polymeric components and the like as well as mixtures thereof.

The membranes of the present invention may be provided with a thickness of between 0.01-10mm. Preferably, the thickness is between 0.1-5mm, and most preferably between 0.5-1.5mm.

The dispenser of the present invention may have membranes of differing properties, by virtue of their chemical make-up and/or thickness, in order to provide the user with a greater variation to alter the passive or boosted emanation rate of the volatile material when the dispenser is in use, as described in more detail hereinafter.

The dispenser may be adapted to be operable, in use, to emanate a wide range of volatile materials, in particular volatile materials such as a fragrance/air freshener, an insecticide, a disinfectant, a bactericide, a fungicide and/or a medicament. In a preferred embodiment the dispenser is adapted such that, in use, it is operable to emanate a fragrance/air freshener.

The volatile material is preferably provided in liquid form.

Preferred liquid fragrance/air fresheners may include a pigment(s) and/or a colourant(s) in order to provide a visual indication to the user of the emanation occurring from the dispenser.

Suitable fragrance/air freshening volatile materials may comprise one or more fragrant components such as cedarwood, oil, sandalwood oil, bergamot, Bulgarian rose oil, patchouli, myrrh, clove leaf oil, linalol, terpineol, menthol, citronellal, and/or phenyl ethyl alcohol.

Further fragrance/air freshening volatile materials that may offer suitable deodorant characteristic include one or more aroma and/or non-aroma chemicals which are known to have an action in reducing the perception of the intensity of malodours. In particular, such suitable materials may include: unsaturated esters, ketones, aldehydes, and/or a fragrant material e.g. citronelial and/or cedarwood oil (which is known to counteract the perception of tobacco malodour).

Further preferred liquid fragrance/air freshening volatile materials may comprise a malodour counteractant and/or an insecticide. Preferably it is the first fragrance composition which may further comprise a malodour counteractant and/or an insecticide.

A suitable insecticide for use in the present invention comprises one or more natural insecticides such as a pyrethroid, nicotinoid, rotenoid and/or one or more synthetic insecticides e.g. Metofluthrin (RTM), Transfluthrin (RTM), Tetramethrin(RTM), Bioallethrin(RTM), Allethrin(RTM), phenthrin, a dinitrophenol, an organothiocyanate, benzene hexachloride, a polychlorinated cyclic hydrocarbon (e.g. Heptachlor(RTM), Aldrin(RTM) and/or Telodrin(RTM)), and/or an organophosphorous (e.g. tetraethyl pyrophosphate).

The fragrance/air freshening volatile materials utilized in the present invention may further comprise an antioxidant such as tocopherol, ascorbyl palmitate, butylated toluene, ascorbic acid, teTt-butyl hydroquinone, beta carotene and/or a gallate. In these volatile materials may optionally comprise a UV stabiliser, such as Uvinol 400.

Turning to the mode of operation of the dispenser. According to the present invention, the dispenser is advantageously operable to passively emanate, in use, a volatile material therefrom, thus without the need of a propellant-powered aerosol or powered automation such as an electric heater, an electric fan or the like. Thus the dispenser of the present invention is capable of emanating, in use, merely by capillary/diffusion action of the volatile material across the membranes and evaporation into the external environment.

In use the dispenser may be orientated such that its side edge(s) are in contact with a surface on which the dispenser is placed. This orientation may permit the membranes to face in a substantially perpendicular direction to the surface. Indeed, this orientation may permit volatile material to be in direct contact with at least one discrete membrane whilst at least one membrane is not in direct contact with said material. The volatile material may then, via capillary/diffusion action, be transported across the membrane(s) which it is in contact with to permit emanation of the material by evaporation from the external surface of the membrane(s).

Whilst the above-described process will permit a continual emanation of the material, the dispenser may, advantageously, have the ability to boost the rate of emanation. The boosting of the emanation rate may be facilitated by a user lifting the dispenser, rotating it and placing back on to the surface such that a substantially opposite side edge(s) of the dispenser is in contact with the surface to the edge(s) that was in contact prior to moving the dispenser. This movement of the dispenser may cause the volatile material to move to a substantially opposite end of the inner chamber of the housing and, in doing so, come into direct contact with at least one membrane that was not in contact with the material prior to this movement. The result of the movement is that for a temporary period of time at least, more than two and possibly all of the membranes will be wetted with volatile material even though at least one membrane is not in direct contact therewith, temporarily permitting more evaporation of the volatile from the exterior surfaces of the membranes. For the membrane(s) that is no longer in direct contact with the volatile material, the quantity of material in these membrane(s) will eventually evaporate away therefrom and, thus, the boosting of the emanation rate will be over until the user moves the dispenser again.

It is considered that the boosting of the rate of emanation may, advantageously, overcome the aforementioned drawbacks of habituation, vacuum build-up and accumulation of volatile material components. Specifically, the movement of the volatile material within the chamber may alleviate any vacuum build-up due to the material moving away from the membrane or membranes suffering such a build-up, thus permitting more throughflow of air. The movement of volatile material may also impart a mixing effect on the components of the material such that the more volatile components are better dispersed throughout the material, thus leading, at least temporarily, to a more uniform emanation profile for all the components of the material.

The boosting of the emanation rate may increase the quantity of material emanating into the environment, thus potentially providing a user with noticeable boost of the material in the surrounding environment, particularly when the volatile material is a fragrance/air freshener, thus reducing the effect of habituation.

The presence of more than two discrete membranes may permit the user of the dispenser to better control the passive/boost emanation rate of the dispenser. In particular, the dispenser is provided with more than two discrete membranes in order for the user to orientate the dispenser such that more or less of discrete membranes are in contact with the volatile material. Differently sized membranes may be provided to facilitate the user in selecting and controlling the passive/boost emanation rate. In such an arrangement a user may expose more discrete membrane(s) to the volatile material when a greater passive or boosted emanation rate is required.

The dispenser may be provided with an access port in the housing to permit the refilling of the level of volatile material. This feature, particularly where fragrances/air fresheners are concerned, may permit a user to alter the odour being emanated without the need to replace the entire dispenser.

The dispenser of the present invention may be configured to be operable for use with a refill of volatile material. The housing may be provided with a section thereof that is movable with respect to the other part or parts of the housing to permit a user access to the inner housing to position a refill therein. The housing may have a section which is hinged or connected by a snap-fit arrangement to allow it to be temporarily opened or removed to permit the loading of the refill.

The housing may be provided with additional sealing means between movable parts of the housing such that, after being opened and subsequently closed, the seal is sufficient to substantially completely prevent liquid and/or gaseous leakage of any volatile material from the inner chamber.

Preferably the side wall of the dispenser where the membranes are located is movable to permit the loading of a refill. This arrangement may be advantageous as a user will be better able to prevent spilling of the volatile material from the refill when loading this into the dispenser. In this arrangement a user would simply need to place the rear wall of the dispenser on a flat surface, remove or open the front wall of the dispenser with the membranes, load the refill into the inner chamber, remove any barrier material holding the volatile material in the refill, replace the front wall of the dispenser ensuring a secure seal with the rest of the housing and position the dispenser on one of its side edges to begin the passive emanation of the volatile material.

The inner surface of the movable part of the housing may be provided with one or more piercing means to further improve the anti-spill properties of the dispenser with a refill is loaded therein. In this arrangement the refill including any barrier material thereon would be loaded into the inner chamber and when the user replaced the movable part of the housing to securely seal it with the rest of the housing, the one or more piercing means would pierce the barrier material to permit the volatile material to contact the membrane(s) of the dispenser.

The refill is preferably shaped to substantially fill the inner chamber of the housing. The refill is preferably provided with a resilient housing having a open side wall covered with a removable or piercable barrier material.

According to a second aspect of the present invention therefore, there is provided method of manually boosting the emanation rate of a dispenser , as defined by method claim 15.

### Brief Description of the Drawings

Embodiments of the invention will now be described, by way of example only, with reference to the following drawings in which:
Fig. 1 illustrates a perspective view of a dispenser;
Fig. 2 illustrates the sequence of movement imparted to the dispenser of Fig. 1 when viewed from the side to activate the boost feature;
Fig. 3 illustrates a perspective view of a dispenser according to the present invention;
Fig. 4 illustrates a perspective view of a further dispenser according to the present invention;
and
Fig. 5 illustrates the varying orientation that may be imparted to the dispenser of Fig. 4 when viewed from the side to vary the passive emanation rate thereof.

### Description of an Example

As can be seen in Fig. 1, a dispenser (10) is illustrated comprising a generally rectangular cube shape defined by a hollow housing with side edges (15) that space apart the front wall and rear wall (not shown) of the dispenser (10). In the front wall of the dispenser (10), are held two discrete membranes (11,12) separated by a separation member (13) of the housing. The membranes (11,12) are secured to the front wall of the housing to form a leak-proof seat therewith. A portion of the internal volume of the housing is filled with a volatile material, preferably a liquid volatile material. In the illustrated example, the volatile material fills the internal volume of the housing up to the dashed line (14). This quantity of volatile material is preferred as it does not directly contact all of the membrane (12), thus likely permitting a general amelioration of vacuum effects as discussed hereinbefore.

Capillary/diffusion effects may cause the volatile material to be carried across the membrane (12) from its surface facing the interior of the housing to its surface facing the exterior of the housing. On reaching the exterior-facing surface of the membrane (12), the volatile material may be emanated into the surrounding environment by evaporation.

Fig. 1 illustrates a first position of the dispenser (10) and it is to be noted that one of the membranes (11) is not in direct contact with the volatile material.

The boost feature according to the example will now be described with reference to Fig. 2 where the movement of the dispenser (10) from a first position (position A) to a second position (position C,D) is demonstrated.

The movement of the dispenser (10) may be provided by a user lifting the dispenser (10), rotating it (position B) and placing back on to the surface such that a substantially opposite side edge(s) of the dispenser is in contact with the surface to the edge(s) that was in contact prior to moving the dispenser (position C,D). This movement of the dispenser may cause the volatile material to move to a substantially opposite end of the interior of the housing and, in doing so, move from direct contact with the membrane (12) and come into direct contact with the other membrane (11). The result of the movement is that for a temporary period of time (as shown in position C) both membranes (11,12) are wetted with volatile material even though only one membrane (11) is in direct contact therewith. With both membranes (11,12) wetted more evaporation of the volatile material from the exterior surfaces of both membranes (11,12) is permitted. For the membrane (12) that is no longer in direct contact with the volatile material, the quantity of volatile material in this membrane(12) will eventually evaporate away therefrom and, thus, once substantially completely evaporated away, the boost period will be over until the user moves the dispenser (10) again.

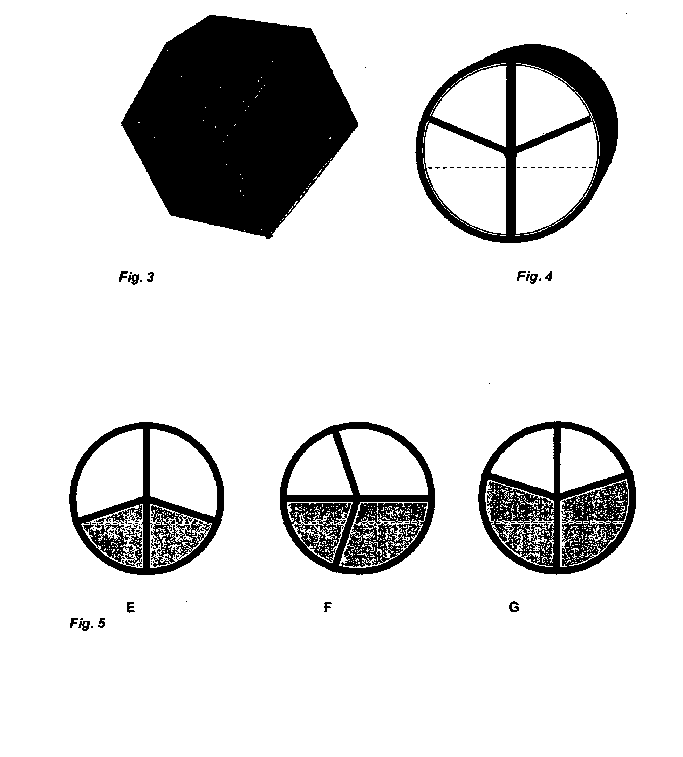

Although the dispenser (10) illustrated in Figs. 1 & 2 is of a general cubical shape, Figs. 3 & 4 illustrate that numerous shapes of housings and membranes have been envisaged within the various embodiments that are in accordance with the present invention.

In Fig.5 the particular orientation of a dispenser in accordance with the present invention illustrates how a user of the dispenser may alter the passive emanation rate of said dispenser.

In the illustrated embodiment, the dispenser is provided with numerous discrete membranes, in this case four membranes with two sets of sizes. If desired however, all of the discrete membranes (or portions of a membrane) could be of different sizes or a combination of sizes. The dashed line illustrates the level of volatile material held within the housing. As can be seen, a user may orientate the housing by altering which part of its side edge is in contact with the surface on which the housing stands. In so altering the orientation, different membranes are placed in direct contact with the volatile material. Where the membranes are of a different size, as is the case in Fig. 5, where the biggest membranes are in direct contact (see G), the greater the passive emanation rate of the volatile material will be. In contrast, where the smaller membranes are in direct contact with the volatile material (see E), the lower the passive emanation rate.

Thus, the embodiment illustrates one example of how a dispenser in accordance with the present invention may be operated to vary the emanation rate either passively by selecting the orientation of the dispenser, or actively by activating the boost feature.

## Claims

1. A passive dispenser for volatile material comprising a housing with an inner chamber configured to hold a quantity of volatile material therein, and further comprising more than two discrete membranes wherein said chamber is configured to permit direct contact between the volatile material and said membranes and wherein, in use, the user controls an emanation rate of the volatile material by orientating the dispenser such that more or less of discrete membranes are in contact with the volatile material and such that at least one of said membranes is not in direct contact with the volatile material.

2. A dispenser according to claim 1, wherein volatile material is provided in the inner chamber.

3. A dispenser according to claim 1 or claim 2, wherein the housing of the dispenser comprises a front wall and a rear wall spaced apart by one or more side edges to define the inner chamber, and wherein the dispenser is configured such that, in use, its operating position is for the side edge or edges of the housing to contact the surface on which the dispenser is placed.

4. A dispenser according to claim 3, wherein the membranes are attached to the front wall thereof, such that one side of each membrane is exposed to the inner chamber of the housing and the other side of the membrane is exposed to the exterior of the housing.

5. A dispenser according to any preceding claim, wherein the dispenser is configured such that, in use, the membranes are not facing toward the surface on which the dispenser is placed.

6. A dispenser according to any preceding claim, wherein the housing has one or more securing means protruding from or mounted on the exterior thereof, wherein said securing means are adapted to engage the surface on which the dispenser is placed.

7. A dispenser according to any preceding claim, wherein a barrier section is located between adjacent membranes.

8. A dispenser according to claim 2 or any claim dependent thereon, wherein the volatile material is present in a quantity in the dispenser that, when in use and the dispenser is orientated in a preferred operating position, the volatile material does not come into direct contact with the entirety of a discrete membrane.

9. A dispenser according to any preceding claim, wherein the membranes have differing properties by virtue of their chemical make-up or thickness to provide the user, in use, with a greater variation to alter the emanation rate.

10. A dispenser according to any preceding claim, wherein membranes of differently sized areas are provided to facilitate the user in selecting and controlling the emanation rate when using the dispenser.

11. A dispenser according to any preceding claim, wherein the dispenser is configured to be operable for use with a refill of volatile material.

12. A dispenser according to claim 11, wherein the housing is provided with a section thereof that is movable with respect to the other part or parts of the housing to permit a user access to the inner housing to position a refill therein, wherein the side wall of the dispenser where the membranes are located is movable to permit the loading of a refill.

13. A dispenser according to claim 12, wherein the inner surface of the movable part of the housing is provided with one or more piercing means.

14. A dispenser according to any of claims 11-13, wherein the refill is shaped to substantially fill the inner chamber of the housing.

15. A method of manually boosting the emanation rate of a dispenser in accordance with claim 2, wherein a volatile material dispenser is manually moved from a first position to a second position such that the volatile material is in direct contact with at least one discrete membrane that said volatile material was not in contact with during said first position, and wherein at least one discrete membrane is not in direct contact with the volatile material in said second position.

## Patentansprüche

1. Passiver Verteiler für flüchtiges Material, der ein Gehäuse mit einer Innenkammer umfasst, welche so ausgebildet ist, dass sie darin eine bestimmte Menge flüchtigen Materials fassen kann, und außerdem mehr als zwei einzelne Membranen umfasst, wobei die Kammer so ausgebildet ist, dass sie direkten Kontakt zwischen flüchtigem Material und den Membranen erlaubt, und wobei der Benutzer bei Gebrauch eine Sprühmenge des flüchtigen Materials reguliert, indem er den Verteiler so ausrichtet, dass mehr oder weniger der einzelnen Membranen mit dem flüchtigen Material in Berührung stehen und so dass mindestens eine der Membranen nicht in direktem Kontakt mit dem flüchtigen Material steht.

2. Verteiler nach Anspruch 1, wobei sich das flüchtige Material in der Innenkammer befindet.

3. Verteiler nach Anspruch 1 oder 2, wobei das Gehäuse des Verteilers eine Vorderwand und eine Rückwand umfasst, die durch eine oder mehrere Seitenkanten voneinander getrennt sind, um die Innenkammer abzugrenzen, und wobei der Verteiler so ausgelegt ist, dass bei Gebrauch in seiner Betriebsposition die Seitenkante oder -kanten des Gehäuses die Oberfläche berühren, auf der der Verteiler angeordnet ist.

4. Verteiler nach Anspruch 3, wobei die Membranen an der Vorderwand desselben angebracht sind, so dass eine Seite jeder Membran der Innenkammer des Gehäuses ausgesetzt ist und die andere Seite der Membran vom Gehäuse nach außen weist.

5. Verteiler nach einem der vorhergehenden Ansprüche, wobei der Verteiler so ausgelegt ist, dass bei Gebrauch die Membranen nicht in Richtung der Oberfläche weisen, auf der der Verteiler angeordnet ist.

6. Verteiler nach einem der vorhergehenden Ansprüche, wobei das Gehäuse eine oder mehr Sicherungsmittel hat, die aus dem Äußeren desselben herausragen oder an der Außenseite desselben befestigt sind, wobei die Sicherungsmittel dafür ausgelegt sind, dass sie in die Oberfläche, auf der der Verteiler angeordnet ist, eingreifen.

7. Verteiler nach einem der vorhergehenden Ansprüche, wobei sich ein Barrierebereich zwischen den benachbarten Membranen befindet.

8. Verteiler nach Anspruch 2 oder einem von diesem Anspruch abhängigen Anspruch, wobei das flüchtige Material im Verteiler in einer solchen Menge vorhanden ist, dass wenn er benutzt wird und der Verteiler in einer bevorzugten Betriebsposition ausgerichtet ist, das flüchtige Material nicht in direkten Kontakt mit einer ganzen einzelnen Membran kommt.

9. Verteiler nach einem der vorhergehenden Ansprüche, wobei die Membranen unterschiedliche Eigenschaften aufgrund ihrer chemischen Zusammensetzung oder Stärke aufweisen, um dem Benutzer bei Gebrauch eine größere Spannbreite zum Ändern der Sprühmenge zu bieten.

10. Verteiler nach einem der vorhergehenden Ansprüche, wobei Membranen mit unterschiedlich großen Flächen bereitgestellt sind, um dem Benutzer das Auswählen und Handhaben der Sprühmenge bei Gebrauch des Verteilers zu erleichtern.

11. Verteiler nach einem der vorhergehenden Ansprüche, wobei der Verteiler so ausgelegt ist, dass er mit einem Nachfüllpack flüchtigen Materials benutzt werden kann.

12. Verteiler nach Anspruch 11, wobei das Gehäuse mit einem Bereich desselben ausgestattet ist, der in Bezug zum anderen Teil oder zu anderen Teilen des Gehäuses beweglich ist, um dem Benutzer Zugang zum Inneren des Gehäuses zu ermöglichen, um darin ein Nachfüllpack anzuordnen, wobei die Seitenwand des Verteilers, wo sich die Membranen befinden, beweglich ist, um das Einlegen eines Nachfüllpacks zu ermöglichen.

13. Verteiler nach Anspruch 12, wobei die innere Fläche des beweglichen Teils des Gehäuses mit einem oder mehreren Durchstechmitteln ausgestattet ist.

14. Verteiler nach einem der Ansprüche 11-13, wobei das Nachfüllpack so geformt ist, dass es die Innenkammer des Gehäuses im Wesentlichen ausfüllt.

15. Verfahren zum manuellen Erhöhen der Sprühmenge eines Verteilers nach Anspruch 2, wobei ein Verteiler für flüchtiges Material manuell von einer ersten Position zu einer zweiten Position bewegt wird, so dass das flüchtige Material mindestens eine einzelne Membran direkt berührt, mit der das flüchtige Material in der ersten Position nicht in Kontakt stand, und wobei mindestens eine einzelne Membran das flüchtige Material in der zweiten Position nicht direkt berührt.

## Revendications

1. Distributeur passif de matériau volatil comprenant un boîtier avec une chambre interne configurée de manière à contenir une quantité de matériau volatil, et comprenant en outre plus de deux membranes discrètes, ladite chambre étant configurée pour permettre un contact direct entre le matériau volatil et lesdites membranes et, pendant l'utilisation, l'utilisateur contrôlant un taux d'émanation du matériau volatil en orientant le distributeur de telle sorte que plus ou moins de membranes discrètes soient en contact avec le matériau volatil et de telle sorte qu'au moins l'une desdites membranes ne soit pas en contact direct avec le matériau volatil.

2. Distributeur selon la revendication 1, dans lequel le matériau volatil est prévu dans la chambre interne.

3. Distributeur selon la revendication 1 ou la revendication 2, dans lequel le boîtier du distributeur comprend une paroi avant et une paroi arrière espacées l'une de l'autre par un ou plusieurs bords latéraux afin de définir la chambre interne, le distributeur étant configuré de telle sorte que, pendant l'utilisation, sa position de fonctionnement soit définie lorsque le ou les bords latéraux du boîtier sont en contact avec la surface sur laquelle est placé le distributeur.

4. Distributeur selon la revendication 3, dans lequel les membranes sont attachées à la paroi avant du distributeur, de telle sorte qu'un côté de chaque membrane soit exposé vers la chambre interne du boîtier et que l'autre côté de la membrane soit exposé vers l'extérieur du boîtier.

5. Distributeur selon l'une quelconque des revendications précédentes, dans lequel le distributeur est configuré de telle sorte que, pendant l'utilisation, les membranes ne soient pas tournées vers la surface sur laquelle est placé le distributeur.

6. Distributeur selon l'une quelconque des revendications précédentes, dans lequel le boîtier présente un ou plusieurs moyens de fixation faisant saillie depuis l'extérieur du boîtier ou étant montés sur l'extérieur du boîtier, lesdits moyens de fixation étant prévus pour s'engager avec la surface sur laquelle est placé le distributeur.

7. Distributeur selon l'une quelconque des revendications précédentes, dans lequel une section formant barrière est située entre des membranes adjacentes.

8. Distributeur selon la revendication 2 ou selon l'une quelconque des revendications dépendantes de celle-ci, dans lequel le matériau volatil est présent dans une certaine quantité dans le distributeur, de telle sorte que, pendant l'utilisation et lorsque le distributeur est orienté dans une position de fonctionnement préférée, le matériau volatil ne vienne pas en contact direct avec la totalité d'une membrane discrète.

9. Distributeur selon l'une quelconque des revendications précédentes, dans lequel les membranes ont des propriétés différentes en raison de leur constitution chimique ou de leur épaisseur, afin de fournir à l'utilisateur, pendant l'utilisation, une plus grande possibilité de faire varier le taux d'émanation.

10. Distributeur selon l'une quelconque des revendications précédentes, dans lequel des membranes de surface de dimensions différentes sont prévues afin de faciliter la sélection et le contrôle par l'utilisateur du taux d'émanation lorsqu'il utilise le distributeur.

11. Distributeur selon l'une quelconque des revendications précédentes, dans lequel le distributeur est configuré de manière à pouvoir fonctionner pour l'utilisation avec une recharge de matériau volatil.

12. Distributeur selon la revendication 11, dans lequel le boîtier est muni d'une section déplaçable par rapport à l'autre partie ou aux autres parties du boîtier afin de permettre à un utilisateur d'accéder au boîtier intérieur afin d'y positionner une recharge, la paroi latérale du distributeur dans laquelle sont disposées les membranes étant déplaçable pour permettre le chargement d'une recharge.

13. Distributeur selon la revendication 12, dans lequel la surface interne de la partie mobile du boîtier est pourvue d'un ou de plusieurs moyens de perçage.

14. Distributeur selon l'une quelconque des revendications 11 à 13, dans lequel la recharge est formée de manière à remplir substantiellement la chambre interne du boîtier.

15. Procédé pour augmenter manuellement le taux d'émanation d'un distributeur selon la revendication 2, dans lequel un distributeur de matériau volatil est déplacé manuellement depuis une première position dans une deuxième position de telle sorte que le matériau volatil soit en contact direct avec au moins une membrane discrète avec laquelle ledit matériau volatil n'était pas en contact dans ladite première position, et dans lequel au moins une membrane discrète n'est pas en contact direct avec le matériau volatil dans ladite deuxième position.
